# EUROPÄISCHE PATENTSCHRIFT

(11) **EP 1 101 096 B1**
(45) Veröffentlichungstag und Bekanntmachung des Hinweises auf die Patenterteilung: **12.02.2003**
(21) Anmeldenummer: 99948704.4
(22) Anmeldetag: 22.07.1999
(51) Int. Cl.: G01N 11/08

(54) **VERFAHREN ZUR BESTIMMUNG WECHSELNDER EISKONZENTRATIONEN IN BINÄREISVOLUMINA**
METHOD OF DETERMINING VARIABLE CONCENTRATIONS OF ICE IN BINARY ICE VOLUMES
PROCEDE POUR DETERMINER DES CONCENTRATIONS VARIABLES DE GLACE DANS DES VOLUMES DE GLACE BINAIRES

(30) Priorität: 01.08.1998 DE 19834781
(43) Veröffentlichungstag der Anmeldung: 23.05.2001
(73) Patentinhaber: Integral Energietechnik GmbH, 24941 Flensburg (DE)
(72) Erfinder: PAUL, Joachim, D-24937 Flensburg (DE)
(74) Vertreter: Biehl, Christian, Dipl.-Phys.
(86) Internationale Anmeldenummer: DE9902307
(87) Internationale Veröffentlichungsnummer: WO00008436

(56) Entgegenhaltungen:
- WO-A-97/48973
- DE-C- 4 325 794
- US-A- 4 653 313
- US-A- 4 888 976
- PATENT ABSTRACTS OF JAPAN vol. 010, no. 232 (P-486), 12. August 1986 (1986-08-12) & JP 61 066143 A (NEC CORP), 4. April 1986 (1986-04-04)

## Beschreibung

Die Erfindung betrifft ein Verfahren zur Bestimmung wechselnder Eiskonzentrationen in Binäreisvolumina. Binäreis, also eine Suspension von kleinen Eiskristallen in einer wässrigen Lösung, bzw. Mischung, wird in vielfältiger Form für Zwecke des Kältetransports, der Kältespeicherung und der Kältenutzung eingesetzt.

Im Gegensatz zu anderen, einphasigen Kühlflüssigkeiten (z. B. Wasser, Sole usw.) ändert sich die Temperatur des Binäreis so lange nicht oder nur unwesentlich, wie Eiskristalle in diesem zweiphasigen Fluid vorhanden sind. Wärmezufuhr drückt sich nicht in einer sensiblen Temperaturerhöhung aus, sondern durch Abschmelzen der Eiskristalle (Latentenergie).

Um die Verwendung von Binäreis steuern zu können, ist nun die Eiskonzentration im Binäreisvolumen zu bestimmen. Dies wird bisher u. a. durch die Messung der elektrischen Leitfähigkeit bzw. des elektrischen Leitwiderstandes sowie Messung des Druckes in einem geschlossenen Speicherbehälter durchgeführt (siehe z.B. DE 43 25 794 C), wobei jeweils nur ein indirekter Rückschluß auf die Eiskonzentration möglich ist, der insbesondere dann nicht ausreicht, wenn die äußeren Rahmenbedingungen nicht konstant bleiben.

So kann z. B. in Systemen, in denen die chemische Zusammensetzung des Binäreisfluides nicht konstant ist, die elektrische Leitfähigkeit kein zuverlässiges Maß für die Eiskonzentration sein. Dasselbe gilt für nicht geschlossene Systeme nicht konstanter Masse, in welchen sich die Änderungen eines Volumens als Änderung der Eiskonzentration ausdrücken würde, was zu Fehlern führt. Die Messung der Dichte des Binäreisfluides ist sehr aufwendig und scheidet aus Kostengründen vor allem für kleinere Anlagen aus. Eine Bestimmung der Viskosität des Binäreisfluides als Maß für die Eiskonzentration ist mit herkömmlichen Methoden nicht möglich, da Binäreis ein Fluid nicht-Newton'schen Typs ist (es ist ein "Bingham fluid") und somit kein verwertbares Signal für die Eiskonzentration liefert.

Auch kalorimetrische Bestimmungen der Eiskonzentration eignen sich lediglich im Labormaßstab, da sie manuelle Maßnahmen erforderlich machen.

Der Erfindung liegt daher die Aufgabe zugrunde, ein Verfahren zu schaffen, mit dem die Eiskonzentration in Binäreisvolumina bestimmt werden kann.

Erfindungsgemäß wird diese Aufgabe durch Verfahren mit den Merkmalen der unabhängigen Ansprüche 1 und 3 gelöst. Die Unteransprüche geben vorteilhafte Ausführungsformen der Erfindung wieder.

Insbesondere ist durch das Verrichten mechanischer Arbeit im zu messenden Volumen durch Fördern des Binäreises mit einer elektrischen Pumpe eine rechnerische Umsetzung möglich, die aus der erfaßten elektrischen Leistung auf die Eiskonzentration zurückschließen läßt.

Zusätzlich bietet sich an, den Pumpendruck, der bei der Förderung von Binäreis in einer Rohrleitung erzeugt wird, zu messen. Dieser Druck steht nicht in Zusammenhang mit dem Druck des Binäreises durch Ausdehnung bei zunehmender Eiskonzentration.

Weitere Vorteile und Merkmale der Erfindung ergeben sich aus nachfolgender Beschreibung eines bevorzugten Ausführungsbeispiels des Verfahrens:

Es wurde festgestellt, daß der benötigte Pumpendruck zur Förderung von Binäreis in einer Rohrleitung oder in einem Behälter in direktem Zusammenhang mit der Eiskonzentration steht. Dieser Druck hat dagegen keinen direkten Zusammenhang mit dem durch Ausdehnung bei zunehmender Eiskonzentration sich erhöhenden Druck.

Bei einer Messung des Drucks des Binäreises nach einer Pumpe, ist dieser Druck ein Maß für die Eiskonzentration, sofern man einen Referenzwert, z. B. bei eisfreiem Betrieb oder bei einer bekannten Eiskonzentration, berücksichtigt. Dasselbe gilt für die Druckdifferenz über eine Strecke. Diese Druckdifferenz kann ebenfalls im Zusammenhang mit der Eiskonzentration gebracht werden.

Damit läßt sich durch die einfache Anbringung zweier Drucksensoren in einer Rohrleitung eine Aussage über den Zustand des Binäreises gewinnen, die dann noch mit einer weiteren Aussage der elektrischen Wirkleistung der Pumpe näher und genauer erfolgen kann.

Bei fluiddynamischen Pumpen (z. B. Kreiselpumpen) sinkt die Fördermenge bei steigendem Gegendruck bzw. bei steigender Druckdifferenz, wie dies bei der Zunahme der Eiskonzentration auftritt. Die sinkende Fördermenge resultiert in einem geringeren Pumpendruck, wobei die steigende Eiskonzentration diesem Absinken entgegenwirkt. Der resultierende Druck ist somit einer Interferenz aus Fördermenge und Eiskonzentration unterworfen.

Bei Verdrängerpumpen (z. B. Zahnradpumpen) bleibt die Fördermenge bei steigendem Gegendruck, bzw. bei steigender Druckdifferenz, wie dies bei der Zunahme der Eiskonzentration auftritt, annähernd konstant (auf jeden Fall deutlich konstanter als bei fluiddynamischen Pumpen). Die Zunahme des Druckes bzw. der Druckdifferenz wird daher bei Verdrängerpumpen noch stärker ausgeprägt sein. Diese eignen sich daher zu einer genaueren Messung.

In einer verzweigten Rohr- und Behälteranordnung, deren Strömungsverhältnisse veränderlich sind (z. B. öffnende oder schließende Armaturen, Umschaltvorgänge, veränderliche (Gegen)Drücke usw.), kann der Druck bzw. die Druckdifferenz zusätzlichen Veränderungen unterworfen sein.

Ungeachtet der vorgenannten Interferenzen und Veränderungen ist es möglich, den durch eine Pumpe aufzubringenden Druck bzw. die herrschende Druckdifferenz in einer Rohrleitung bzw. in einem Behälter soweit von Nebeneinflüssen zu isolieren, daß die Veränderung des Drucks bzw. der Druckdifferenz als Maß für die Eiskonzentration herangezogen werden kann. Ggf. ist eine Kalibrierung vorzunehmen. Hierbei wird man vorteilhafterweise die Druck(differenz)messung an solchen Meßorten vornehmen, deren Anordnung, Geometrie und Ausführung vereinheitlicht sind, so daß eine werkseitige Meßortdefinition gegeben ist. Dies sind z. B. der Ein- und/oder Austritt aus dem Eiserzeuger oder eine definierte Rohrstrecke.

Da der Druck als Maß der Pumpenleistung direkt proportional der elektrischen Wirkleistung der Pumpe ist, kann auch diese als Maß für die Eiskonzentration herangezogen werden, sofern die geometrischen Verhältnisse nach der Pumpe vereinheitlicht sind.

## Patentansprüche

1. Verfahren zur Bestimmung wechselnder Eiskonzentration in Binäreisvolumina, **gekennzeichnet durch**
- Verrichten mechanischer Arbeit im zu messenden Volumen mit einer das Binäreisfluid fördernden Pumpe,
- Erfassen der von der Pumpe erbrachten elektrischen Leistung, und
- rechnerische Umsetzung der erfaßten elektrischen Leistung in ein Maß für die Eiskonzentration.

2. Verfahren nach Anspruch 1, **dadurch gekennzeichnet, daß** über eine Druckmessung die von einer zu überwachenden elektrischen, das Binäreisfluid fördernden Pumpe geleistete elektrische Arbeit bestimmt wird.

3. Verfahren zur Bestimmung wechselnder Eiskonzentration in Binäreisvolumina, **gekennzeichnet durch**
- Verrichten mechanischer Arbeit am zu messenden Volumen mit einer das Binäreisfluid fördernden Pumpe,
- Erfassen des bei der Förderung des Binäreisfluids erzeugten Pumpendruckes, und
- rechnerische Umsetzung des erfaßten Drucks in ein Maß für die Eiskonzentration.

4. Verfahren nach Anspruch 3, **dadurch gekennzeichnet, daß** Erfassen des Drucks mit zwei Drucksensoren in einer Rohrleitung nach der Punpe erfolgt.

## Claims

1. Method for determining changing ice concentrations in binary ice volumes, **characterized by**
- carrying out mechanical work in the volume to be measured with a pump delivering the binary ice fluid,
- determining the electrical power carried out by the pump, and
- mathematical conversion of the determined electrical power into a measure for the ice concentration.

2. Method according to claim 1, **characterized in that** by means of a pressure measurement determination takes place of the electrical work carried out by an electric, binary ice fluid - delivering pump to be monitored.

3. Method for determining changing ice concentrations in binary ice volumes **characterized by**:
- carrying out electrical work in the volume to be measured with a pump delivering the binary ice fluid,
- determining the pump pressure produced when delivering the binary ice fluid,
- mathematical conversion of the determined pressure in a measure for the ice concentration.

4. Method according to claim 3, **characterized in that** the determination of the pressure is carried out with two pressure sensors in a pipe behind the pump.

## Revendications

1. Procédé destiné à déterminer la concentration variable de glace dans des volumes de glace binaire, **caractérisé par**
l'exécution d'un travail mécanique dans le volume à mesurer avec une pompe déplaçant le fluide de glace binaire,
l'enregistrement de la puissance électrique fournie par la pompe, et
la conversion par calcul de la puissance électrique enregistrée en une valeur pour la concentration de glace.

2. Procédé selon la revendication 1, **caractérisé en ce que** le travail électrique fourni par une pompe électrique à contrôler, déplaçant le fluide de glace binaire, est déterminé par l'intermédiaire d'une mesure de la pression.

3. Procédé destiné à déterminer la concentration variable de glace dans des volumes de glace binaire, **caractérisé par**
l'exécution d'un travail mécanique dans le volume à mesurer avec une pompe déplaçant le fluide de glace binaire,
l'enregistrement de la pression de la pompe générée lors du déplacement du fluide de glace binaire, et
la conversion par calcul de la pression enregistrée en une valeur pour la concentration de glace.

4. Procédé selon la revendication 3, **caractérisé en ce que** la pression est enregistrée au moyen de deux capteurs de pression dans une canalisation en aval de la pompe.
